(19) **Europäisches Patentamt**

**European Patent Office**

**Office européen des brevets**

(11) Veröffentlichungsnummer: **0 310 827 B1**

(12) **EUROPÄISCHE PATENTSCHRIFT**

(45) Veröffentlichungstag der Patentschrift:
31.10.90

(51) Int. Cl.⁵: **C07B 63/02**

(21) Anmeldenummer: **88114909.0**

(22) Anmeldetag: **13.09.88**

(54) **Verfahren zum Entfernen von Metallhalogeniden aus flüssigen, mit Wasser nicht mischbaren organischen Stoffen.**

(30) Priorität: **23.09.87 DE 3731995**

(43) Veröffentlichungstag der Anmeldung:
**12.04.89 Patentblatt 89/15**

(45) Bekanntmachung des Hinweises auf die Patenterteilung:
**31.10.90 Patentblatt 90/44**

(84) Benannte Vertragsstaaten:
**DE FR GB IT**

(56) Entgegenhaltungen:
**Keine Entgegenhaltungen**

(73) Patentinhaber: **BAYER AG,
D-5090 Leverkusen 1 Bayerwerk(DE)**

(72) Erfinder: **Hartung, Sigurd, Dr., verstorben(DE)**
Erfinder: **Beck, Uwe, Dr., v.-Diergardt-Strasse 60,
D-5090 Leverkusen 1(DE)**
Erfinder: **Kappler, Ulrich, Dr., Rheindorfer Strasse 163c,
D-4018 Langenfeld(DE)**
Erfinder: **Mitschker, Alfred, Dr., Am Gartenfeld 50,
D-5068 Odenthal-Holz(DE)**
Erfinder: **Gräfe, Andreas, Odenthaler Markweg 60,
D-5060 Bergisch-Gladbach(DE)**

**Beschreibung**

Die Erfindung betrifft ein Verfahren zum Entfernen von Metallhalogeniden aus flüssigen, mit Wasser nicht mischbaren organischen Stoffen.

Bei der Herstellung organischer Verbindungen fallen häufig Lösungen oder Gemische organischer Verbindungen oder auch einzelne organische, mit Wasser nicht mischbare Verbindungen an, die noch kleine Mengen an Metallhalogeniden, z.B. $AlCl_3$, $TiCl_4$, $SnCl_4$, $ZnCl_2$, $MnCl_2$, $MoCl_3$, $SbCl_3$, $SbCl_5$, $ZrCl_4$ oder $FeCl_3$ ($Br_3$) enthalten, und die von diesen Metallhalogeniden befreit werden müssen.

So fallen z.B. bei der Chlorierung organischer Verbindungen, wie Benzol, Toluol, Nitrobenzol oder Naphthalin in Gegenwart von Eisenhalogeniden, z.B. von Fe-(III)-chlorid, Eisenhalogenid-haltige Chlorierungsgemische an. Aus diesen Gemischen müssen die gewünschten reinen Ver bindungen meist durch fraktionierte Destillation gewonnen werden. Vor der Destillation müssen die Eisenhalogenide jedoch aus den Gemischen vollständig entfernt werden, weil sie bei der destillativen Aufarbeitung stören; sie führen zu Verstopfungen und zur Korrosion in den Destillationskolonnen.

Die bislang zum Entfernen von Eisenhalogeniden aus Chlorierungsgemischen angewandten Verfahren, Waschen der Chlorierungsgemische mit Wasser oder Zusatz trockener Soda zu den Gemischen, haben den Nachteil, daß bei ihnen erhebliche Mengen an Abwasser bzw. feste meist schlecht filtrierbare Abfallprodukte anfallen.

Es wurde nun gefunden, daß sich die Eisenhalogenide auf einfache Weise, ohne daß Abwasser und/oder Abfallprodukte anfallen, aus den Chlorierungsgemischen entfernen lassen, wenn man diese Gemische mit wasserhaltigen Anionenaustauschern behandelt.

Überraschenderweise wurde gefunden, daß wasserhaltige Anionenaustauscher nicht nur Halogenid, sondern auch Eisenionen binden. Es wurde gefunden, daß die Eisenionen nicht, wie dies nach dem Stand der Technik für die Adsorption von Metallionen durch Ionenaustauscher zu erwarten war, von starksauren Kationenaustauschern, sondern überraschenderweise von Anionenaustauschern gebunden werden, wenn diese einen gewissen Wassergehalt aufweisen.

Zum Stand der Technik für die Adsorption von Ionen aus wässrigen Lösungen oder organischen Lösungsmitteln mittels Ionenaustauschern gehört, daß Kationen, z.B. Metallionen, durch Kationenaustauscher und Anionen, z.B. in Form von Anionenkomplexen vorliegende Metallionen, durch Anionenaustauscher gebunden werden (siehe Ullmanns Encyclopädie der Technischem Chemie, 4. Aufl. Bd 13, S. 279 - 346 und Industrial and Engineering Chemistry Vol. 45, S. 2577 - 2580 (1953)). Da das Eisen in den Chlorierungsgemischen jedoch nicht in Form eines Anionenkomplexes vorliegt - der bei der Chlorierung entstehende Chlorwasserstoff wird durch Ausblasen der Chlorierungsmischung entfernt - bestand keine Veranlassung zu der Annahme, daß die Eisenionen, wo sie durch einen Kationenaustauscher nicht zu entfernen waren, nunmehr durch einen Anionenaustauscher gebunden werden könnten.

Es wurde weiterhin gefunden, daß Anionenaustauscher mit einem gewissen Wassergehalt sich nicht nur zum Entfernen von Eisenhalogeniden, wie Eisenchlorid und -bromid aus Chlorierungsgemischen, sondern ganz allgemein dazu eignen, Metallhalogenide wie die als Chlorierungskatalysatoren verwendeten Metallhalogenide $FeCl_3$, $FeBr_3$, $AlCl_3$, $TiCl_4$, $SnCl_4$, $ZnCl_2$, $SbCl_3$, $SbCl_5$, $MnCl_2$, $MoCl_3$, $ZrCl_4$ aus flüssigen, mit Wasser nicht mischbaren organischen Stoffen zu entfernen.

Die Erfindung betrifft daher ein Verfahren zum Entfernen von Metallhalogeniden, vorzugsweise von als Chlorierungskatalysatoren verwendeten Metallhalogeniden, insbesondere von Eisenhalogeniden aus flüssigen, mit Wasser nicht mischbaren organischen Stoffen; das Verfahren ist dadurch gekennzeichnet, daß man die die Metallhalogenide enthaltenden, flüssigen, mit Wasser nicht mischbaren organischen Stoffe mit wasserhaltigen Anionenaustauschern behandelt.

Aus wirtschaftlichen Gründen eignet sich das Verfahren besonders zum Entfernen kleiner Mengen Metallhalogenid, z.B. Mengen < 200 ppm.

Der Wassergehalt der Anionenaustauscher sollte 40 bis 80 Gew.-%, vorzugsweise 50 bis 70 Gew.-%, bezogen auf das Gesamtgewicht des feuchten Anionenaustauschers betragen.

Als Anionenaustauscher können grundsätzlich alle bekannten Anionenaustauschertypen, d.h. makroporöse und gelförmige, starkbasische und schwachbasische, Anionenaustauscher mit einer Matrix aus vernetztem Polystyrol oder mit einer Matrix aus vernetztem Polyacrylat verwendet werden. Besonders bewährt haben sich jedoch makroporöse Anionenaustauscher mit einer Matrix von vernetztem Polystyrol. Diese Anionenaustauscher können stark oder schwach basisch sein.

Die Anionenaustauscher werden in ihrer OH-Form eingesetzt.

Das erfindungsgemäße Verfahren wird vorzugsweise so ausgeführt, daß man den vom Metallhalogenid zu befreienden flüssigen, mit Wasser nicht mischbaren Stoff durch den in einer geeigneten Vorrichtung angeordneten Anionenaustauscher fließen läßt. Eine geeignet Vorrichtung ist z.B. ein Filterrohr, das an einem oder an beiden Enden mit für Flüssigkeiten durchlässigen und für Anionenaustauscher undurchlässigen Boden versehen ist. Die spezifische Belastung, mit der der flüssige organische Stoff über den Anionenaustauscher filtriert wird, sollte etwa 2 bis 10 Bettvolumina (BV)/h betragen

$$BV = \frac{m^3 \; \text{Flüssigkeit}}{m^3 \; \text{Anionenaustauscher}}.$$

Die zu reinigende organische Verbindung wird solange über den Anionenaustauscher geleitet, bis die Konzentration der Metall- und Halogenidionen in dem aus dem Anionenaustauscher ablaufenden flüssigen Stoff auf den als Grenzwert vorgegebenen Wert angestiegen ist.

Nach dem Erreichen des vorgegebenen Grenzwertes im Ablauf des Anionenaustauschers wird das Überleiten des zu reinigenden flüssigen organischen Stoffes über den Anionenaustauscher beendet. Der verbrauchte Anionenaustauscher kann anschließend nach Behandeln mit verdünnter wäßriger Salzsäure, z.B. 20 %iger Salzsäure, und Regenerieren in an sich bekannter Weise mit 2 -10 %iger Natronlauge erneut eingesetzt werden.

Die erfindungsgemäß zu behandelnden flüssigen, mit Wasser nicht mischbaren organischen Stoffe müssen nicht bei Raumtemperatur flüssig sein, sondern es genügt, wenn sie bei Temperaturen unterhalb von 100°C flüssig sind, denn die Adsorption der Metallhalogenide aus den organischen Stoffen wird durch erhöhte Temperaturen nicht beeinträchtigt.

Die erfindungsgemäße Behandlung der flüssigen, mit Wasser nicht mischbaren organischen Stoffe kann sowohl bei Raumtemperatur als auch bei erhöhten Temperaturen von 20 bis 100°C vorgenommen werden. Die Behandlungstemperatur richtet sich nach der Temperatur, bei der der organische Stoff in flüssigem Zustand vorliegt, sie richtet sich aber auch danach, mit welcher Temperatur der zu behandelnde organische Stoff anfällt und ob und wenn ja, bei welcher Temperatur er nach dem Entfernen des Metallhalogenids weiter verarbeitet werden soll. Das bei der Chlorierung von Toluol in Gegenwart von Eisen-(III)-chlorid oder -bromid erhaltene, durch Ausblasen mit Luft vom gelösten Chlorwasserstoff befreite Chlorierungsgemisch weist eine Temperatur von etwa 55°C auf. Es kann ohne abzukühlen unmittelbar über den Anionenaustauscher geleitet und anschließend unmittelbar destilliert werden. Bei dieser Arbeitsweise werden unnötige Energieverluste vermieden, wie sie beim Abkühlen des Chlorierungsgemisches auf Raumtemperatur und erneutes Erwärmen des Gemisches auf die Destillationstemperatur zwangsläufig eintreten.

Als in dem erfindungsgemäßen Verfahren einsetzbare flüssige, mit Wasser nicht mischbare organische Stoffe seien beispielsweise genannt:

Aromatische Kohlenwasserstoffe wie Benzol, Toluol, Xylol, Ethylbenzol und Naphthalin; chlorierte aromatische Kohlenwasserstoffe wie Chlorbenzol, Dichlorbenzol, Polychlorbenzole; aliphatische Kohlenwasserstoffe wie Heptan, Cyclohexan; chlorierte aliphatische Kohlenwasserstoffe wie Chloroform, Tetrachlormethan, Dichlorethan; nitrierte aromatische Kohlenwasserstoffe wie Nitrobenzol oder Nitrotoluole; nitrierte aliphatische Kohlenwasserstoffe wie Nitromethan; chlorierte nitrierte aromatische Kohlenwasserstoffe wie Nitrochlorbenzole, Nitrochlortoluole.

In dem erfindungsgemäßen Verfahren werden bevorzugt als flüssige, mit Wasser nicht mischbare organische Stoffe Chlorierungsgemische eingesetzt, wie sie bei der Chlorierung von Benzol, Chlorbenzol, Toluol, Chlortoluol, Benzoylchlorid und Naphthalin in Gegenwart von Eisen-III-chlorid und/oder Eisen-III-bromid anfallen. Diese Chlorierungsgemische werden zunächst durch Ausblasen mit Luft oder Stickstoff weitgehend von dem in ihnen gelösten Chlorwasserstoff befreit, before sich nach dem erfindungsgemäßen Verfahren vom Eisenhalogenid befreit werden.

Beispiel 1

Ein Chlorierungsgemisch, das bei der Chlorierung von Toluol in Gegenwart von Eisen-(III)-chlorid als Katalysator erhalten und anschließend durch 10-stündiges Ausblasen mit Luft vom gelösten Chlorwasserstoff befreit worden war (Zusammensetzung des Gemisches: 60 Gew.-% Toluol, 20,5 Gew.-% o-Chlortoluol, 0,3 Gew.-% m-Chlortoluol, 18,7 Gew.-% p-Chlortoluol, 0,4 Gew.-% Dichlortoluole; Gehalt an $Fe^{3+}$: 173 ppm, $Cl^-$: 350 ppm), wird mit einer spezifischen Belastung von 4 Bettvolumina (BV)/h von oben nach unten über 500 ml eines in einem senkrecht angeordneten Filterrohr (Durchmesser: 45 mm) befindlichen makroporösen, mittelbasischen, wasserfeuchten Anionenaustauschers auf Basis von mit 6 Gew.-% Divinylbenzol vernetztem Polystyrol (Totalkapazität an basischen Gruppen: 1,5 Mol/l, davon 0,3 Mol/l stark basische Gruppen; Wassergehalt: 60 Gew.-%) geleitet. Während des Durchleitens ist der Anionenaustauscher vom Chlorierungsgemisch bedeckt.

Der Gehalt des aus dem Anionenaustauscher ablaufenden Chlorierungsgemisches an Eisen- und Chloridionen beträgt:

$Fe^{3+}$ : < 1 ppm;
$Cl^-$ : <32 ppm.

Nach einem Durchsatz von 79 l steigt der Eisenchloridgehalt in Ablauf des Anionenaustauschers merklich an, der Anionenaustauscher ist erschöpft. Das Durchleiten wird beendet und der Anionenaustauscher zunächst mit 20 %iger wäßriger Salzsäure behandelt, und anschließend in üblicher Weise mit verdünnter Natronlauge (2 -10%ig) regeneriert.

Praktisch das gleiche Ergebnis (Gehalt des aus dem Anionenaustauscher ablaufenden Chlorierungsgemisches an $Fe^{3+}$ und $Cl^-$ und Durchsatz bis zur Erschöpfung des Anionenaustauschers) wurde erreicht als das Chlorierungsgemisch beim Überleiten über den Anionenaustauscher eine Temperatur von 55 ° C aufwies.

Beispiel 2

Es wurde wie im Beispiel 1 beschrieben verfahren, nur wurde ein Chlorierungsgemisch verwendet, das nur 82 ppm $Fe^{3+}$ und 160 ppm $Cl^-$ enthielt.

Der Gehalt des aus dem Anionenaustauscher ablaufenden Chlorierungsgemisches an Eisen- und Chloridionen betrug:

$Fe^{3+}$: 1 ppm;
$Cl^-$ : 25 ppm.

Nach Durchleiten von 192 l Chlorierungsgemisch war der Anionenaustauscher erschöpft.

Beispiel 3

Das in Beispiel 1 beschriebene Chlorierungsgemisch wird mit einer spezifischen Belastung von 4 BV/h über 500 ml eines makroporösen schwachbasischen wasserfeuchten Anionenaustauschers (Matrix: mit 8 % Divinylbenzol vernetztes Polystyrol; Totalkapazität an basischen Gruppen: 2,0 Val/l; Wassergehalt 50 Gew. %) geleitet.

Gehalt des Chlorierungsgemisches an Eisen- und Chloridionen vor der Behandlung mit dem Anionenaustauscher: $Fe^{3+}$: 75 ppm; $Cl^-$: 150 ppm.

Gehalt des aus dem Anionenaustauscher ablaufenden Chlorierungsgemisches an Eisen- und Chloridionen:

$Fe^{3+}$: 1 ppm;
$Cl^-$ : 51 ppm.

Nach Durchleiten von 212 l Chlorierungsgemisch ist der Anionenaustauscher erschöpft.

Beispiel 4

Es wird wie in Beispiel 1 beschrieben gearbeitet, nur wird ein anderes Chlorierungsgemisch eingesetzt (Zusammensetzung des Chlorierungsgemisches: 35 Gew.-% o-Chlortoluol, 19,2 Gew.-% 2,5-Dichlortoluol, 16,9 Gew.-% 2,6-Dichlortoluol, 11,1 Gew.-% 2,4-Dichlortoluol, 8,3 Gew.-% 2,3-Dichlortoluol, 10,3 Gew.-% Trichlortoluole; Gehalt des Gemisches an Eisen- und Chloridionen: $Fe^{3+}$: 12 ppm; $Cl^-$: 25 ppm). Das Chlorierungsgemisch wird mit einer spezifischen Belastung von 2 BV/h über den Anionenaustauscher geleitet.

Der Gehalt des aus dem Anionenaustauscher abfließenden Chlorierungsgemisches an Eisen- und Chloridionen beträgt:

$Fe^{3+}$: < 1 ppm;
$Cl^-$ : <10 ppm.

Nach Durchsatz von 458 l Chlorierungsgemisch ist der Anionenaustauscher erschöpft.

Beispiel 5

Ein Chlorierungsgemisch, das bei der Chlorierung von Naphthalin in Gegenwart von Fe-(III)-chlorid als Katalysator erhalten worden war (Zusammensetzung des Gemisches: 30 Gew.-% Naphthalin, 65 Gew.-% Mono-Chlornaphthalin, 5 Gew.-% Dichlornaphthalin), wird bei einer Temperatur von 60 ° C von oben nach unten über 500 ml eines makroporösen schwachbasischen, wasserfeuchten Anionenaustauschers (Matrix: mit 8 % Divinylbenzol vernetztes Polystyrol; Totalkapazität an basischen Gruppen: mind. 1,8 Val/l, Wassergehalt: 45 - 55 Gew.-%) mit einer spezifischen Belastung von 4 BV/h geleitet; der Anionenaustauscher ist in einem senkrecht angeordneten Filterrohr (Durchmesser 45 mm) untergebracht.

Gehalt des Chlorierungsgemisches an Eisen- und Chloridionen vor der Behandlung: $Fe^{3+}$: 280 ppm; $Cl^-$: 550 ppm; Gehalt des aus dem Anionenaustauscher ablaufenden Chlorierungsgemisches an Eisen- und Chloridionen:

$Fe^{3+}$: 40 ppm;
$Cl^-$ : 100 ppm.

Nach Durchsatz von 50 l Chlorierungsgemisch ist der Anionenaustauscher erschöpft.

Patentansprüche

1. Verfahren zum Entfernen von Metallhalogeniden aus flüssigen, mit Wasser nicht mischbaren organischen Stoffen, dadurch gekennzeichnet, daß man die die Metallhalogenide enthaltenden, flüssigen, mit Wasser nicht mischbaren organischen Stoffe mit einem wasserhaltigen Anionenaustauscher in der OH-Form behandelt.

2. Verfahren gemäß Anspruch 1, dadurch gekennzeichnet, daß der wasserhaltige Anionenaustauscher 40 bis 80 Gew.-% Wasser, bezogen auf das Gesamtgewicht des wasserfeuchten Anionenaustauschers enthält.

3. Verfahren gemäß Anspruch 1 oder 2, dadurch gekennzeichnet, daß der Anionenaustauscher ein makroporöser, stark- oder schwachbasischer Anionenaustauscher auf Basis von vernetztem Polystyrol ist.

4. Verfahren gemäß einem der Ansprüche 1 bis 3, dadurch gekennzeichnet, daß es zum Entfernen von Metallhalogeniden aus Chlorierungsprodukten angewendet wird, wie sie bei der Chlorierung aromatischer Kohlenwasserstoffe in gegenwart von Metallhalogenid-Chlorierungskatalysatoren anfallen.

5. Verfahren gemäß einem der Ansprüche 1 bis 4, dadurch gekennzeichnet, daß es zum Entfernen von Eisenhalogeniden angewendet wird.

6. Verfahren gemäß einem der Ansprüche 1 bis 4, dadurch gekennzeichnet, daß es zum Entfernen von Eisen-III-chlorid oder Eisen-III-bromid angewendet wird.

Claims

1. Process for removing metal halides from liquid organic water-immiscible substances, characterized in that the liquid organic water-immiscible substances which contain the metal halides are treated with a watercontaining anion-exchanger in the OH form.

2. Process according to Claim 1, characterized in that the water-containing anion-exchanger contains 40 to 80% by weight of water, based on the total weight of the water-moist anion-exchanger.

3. Process according to Claim 1 or 2, characterized in that the anion-exchanger is a macroporous, strongly or weakly basic anion-exchanger based on crosslinked polystyrene.

4. Process according to one of Claims 1 to 3, characterized in that it is used for removing metal halides from chlorination products, such as are formed in the chlorination of aromatic hydrocarbons in the presence of metal halide chlorination catalysts.

5. Process according to one of Claims 1 to 4, characterized in that it is used for removing iron halides.

6. Process according to one of Claims 1 to 4, characterized in that it is used for removing iron (III) chloride or iron (III) bromide.

**Revendications**

1. Procédé pour éliminer des halogénures métalliques de matières organiques liquides non miscibles à l'eau, caractérisé en ce qu'on traite les matières organiques liquides non miscibles à l'eau contenant les halogénures métalliques avec un échangeur anionique contenant de l'eau, sous la forme OH.

2. Procédé suivant la revendication 1, caractérisé en ce que l'échangeur anionique contenant de l'eau renferme 40 à 80% en poids d'eau par rapport au poids total d'échangeur anionique humecté d'eau.

3. Procédé suivant la revendication 1 ou 2, caractérisé en ce que l'échangeur anionique est un échangeur anioniqne macroporeux fortement ou faiblement basique, à base de polystyrène réticulé.

4. Procédé suivant l'une des revendications 1 à 3, caractérisé en ce qu'il est appliqué à l'élimination d'halogénures métalliques de produits de chloration tels qu'on en obtient dans la chloration d'hydrocarbures aromatiques en présence de catalyseurs de chloration à base d'halogénures métalliques.

5. Procédé suivant l'une des revendications 1 à 4, caractérisé en ce qu'il est appliqué à l'élimination d'halogénures de fer.

6. Procédé suivant l'une des revendications 1 à 4, caractérisé en ce qu'il est appliqué à l'élimination du chlorure de fer-III ou du bromure de fer-III.